# EUROPÄISCHE PATENTSCHRIFT

(11) **EP 1 265 986 B1**
(45) Veröffentlichungstag und Bekanntmachung des Hinweises auf die Patenterteilung: **24.02.2016**
(21) Anmeldenummer: 01913858.5
(22) Anmeldetag: 09.03.2001
(51) Int. Cl.: C12N 5/02, A61K 35/32, A61P 19/00

(54) **VERFAHREN ZUR IN VITRO-HERSTELLUNG VON DREIDIMENSIONALEM, VITALEM KNORPEL- ODER KNOCHENGEWEBE**
METHOD FOR IN VITRO PRODUCTION OF THREE-DIMENSIONAL VITAL CARTILAGE OR BONE TISSUE
PROCEDE PERMETTANT DE PRODUIRE I IN VITRO /I DU TISSU CARTILAGINEUX OU OSSEUX TRIDIMENSIONNEL VIVANT

(30) Priorität: 13.03.2000 DE 10013223
(43) Veröffentlichungstag der Anmeldung: 18.12.2002
(73) Patentinhaber: Co.Don Aktiengesellschaft, 14513 Teltow (DE)
(72) Erfinder: LIBERA, Jeanette, 13051 Berlin (DE); ANDERER, Ursula, 12529 Berlin (DE); FRITSCH, Karl-Gerd, 14195 Berlin (DE); JOSIMOVIC-ALASEVIC, Olivera, 10717 Berlin (DE)
(74) Vertreter: Kilger, Ute
(86) Internationale Anmeldenummer: PCT/EP2001/002698
(87) Internationale Veröffentlichungsnummer: WO 2001/068811

(56) Entgegenhaltungen:
- US-A- 5 723 331
- FRANCHIMONT P ET AL: "EFFECTS OF HORMONES AND DRUGS ON CARTILAGE REPAIR" JOURNAL OF RHEUMATOLOGY, Bd. 16, Nr. SUPPL. 18, 1989, Seiten 5-9, XP001016321 ISSN: 0315-162X
- FRITSCH KG ET AL: "Advances in Autologous Chondrocyte Transplantation (ACT)" TECHVEST'S FIRST ANNUAL CONFERENCE ON TISSUE REPAIR, REPLACEMENT AND REGENERATION, OCTOBER 27-28, 1999, [Online] XP002176591 New York Marriot East Side Hotel, New York Gefunden im Internet: <URL:www.techvestllc.com/conference/abstra cts/codon_abstract.htm> [gefunden am 2001-09-04]
- LÖHNERT J ET AL: "Autologe Chondrozytentransplantation (ACT) im Kniegelenk; Erste klinische Ergebnisse" ARTHROSKOPIE, Bd. 12, 1999, Seiten 34-42, XP002176592
- GRUBER R ET AL: "UNTERSUCHUNGEN ZUR IN-VITRO-KULTIVIERUNG VON HUMANCHONDROZYTEN BEI EINSATZ VON AUTOLOGEM HUMAN-SERUM ALS MEDIUMSUSATZ: MINIMIERUNG DESMOEGLICHEN RISIKOS EINER INFEKTION MIT ERREGERN VON PRIONEN-ERKRANKUNGEN ANALYSIS OF THE IN VITRO ENGINEERING OF HUMAN CHONDROCYTES WITH FCS-FREE CULTURE-MEDIUM MINIM", LARYNGO-RHINO-OTOLOGIE, THIEME, STUTTGART, DE, DE, vol. 75, no. 2, 1 February 1996 (1996-02-01), pages 105-108, XP008000698, ISSN: 0935-8943

## Beschreibung

Die Erfindung betrifft ein äußerst einfaches Verfahren zur in vitro-Herstellung von einem Zellaggregat aus dreidimensionalem, vitalem und mechanisch stabilem Knorpel- oder Knochengewebe und dessen Verwendung als Transplantationsmaterial zur Behandlung von Knorpel- oder Knochendefekten und von degenerativen Erkrankungen, wie z.B. Rheuma oder Arthrose sowie dessen Verwendung zur Testung von Wirkstoffen und physikalischen Faktoren. Erfindungsgemäß können die verschiedensten Knorpel- und Knochengewebe hergestellt werden, so z.B. hyaline, elastische, Faser- oder Bindegewebsknorpel wie beispielsweise Gelenksknorpel, Nasenknorpel, Meniskusknorpel oder Bandscheibenknorpel.

Auf dem Gebiet des Tissue Engineering wird seit längerem nach Lösungen zum Aufbau körpereigenen Gewebes gesucht. Dazu werden zum einen körpereigene Zellen mit und ohne Trägermaterial verwendet und zum anderen ausschließlich Trägermaterialien in den Defekt eingebracht, wobei je nach Indikation resorbierbare oder nicht resorbierbare Materialien verwendet werden können.

Bei der Verwendung von Trägermaterialien ist nachteilig, dass deren Abbauprodukte andere Gewebe schädigen können und dass bei Verwendung von nicht autogenen, also nicht patienteneigenen Trägermaterialien Immunreaktionen oder Infektionen mit tierischen oder menschlichen Erregern auftreten können.

Eine bekannte Methode unter Verwendung von körpereigenen Zellen ist die Knorpel- und Knochenzelltransplantation, die zur Behandlung von Knorpel- und Knochendefekten angewandt wird. Dabei wird das Potential der Knorpel- und Knochenzellen genutzt, um *in vivo* neues Gewebe aufzubauen. So werden beispielsweise dem Patienten Knorpel- oder Knochenbiopsien entnommen, daraus Knorpel- oder Knochenzellen isoliert, mittels Zellkultivierung vermehrt und anschließend dem Patienten die Zellen im Bereich des Gewebedefektes, z.B. durch Einspritzen, transplantiert. Dort bilden sie neues Gewebe und füllen somit den Defekt vollständig auf.

Durch die genannten Verfahren wird erreicht, dass nach Applikation der Zelltransplantate oder Einbringen der Trägermaterialien im Körper Gewebe aufgebaut wird.

Ein weiteres Ziel im Tissue Engineering besteht jedoch darin, körpereigenes Gewebe bereits *in vitro* vorzufertigen. Dazu sind aus der Literatur eine Vielzahl von Verfahren bekannt (vgl. DE 195 40 487, WO 97/46665, DE 197 52 900, US 5.932.459), die entweder spezielle Vorrichtungen oder Träger benötigen, viele Verfahrensschritte beinhalten oder die Zugabe von wachstumsfördernden Verbindungen, die Fremdstoffe für den Körper darstellen, erforderlich machen. Aufgabe der vorliegenden Erfindung war es deshalb, ein möglichst einfaches Verfahren zur Herstellung von typischem Knorpel- oder Knochengewebe bereitzustellen, mit dem vitales, dreidimensionales und mechanisch stabiles Gewebe hergestellt werden kann, das zur Transplantation geeignet ist und ein schnelles Anwachsen im Körper bzw. ein schnelles Auffüllen des Knorpel- oder Knochendefektes gewährleistet. Außerdem soll das in vitro hergestellte Gewebe möglichst keine immunologischen Reaktionen im Organismus, der das Transplantat erhält, auslösen.

US 5,723,331 offenbart zylinderförmige Zellaggregate, die in unterschiedlich vorgeformten wells, je nach Bedarf, gebildet werden. Die vorgeformten wells bestehen aus ebenen Böden. Entsprechend werden ebene Knorpeltransplantate gebildet, welche makrochirurgisch in den Wundgrund integriert werden können. Gemäß US 5,723,331 wird ausschließlich mit xenogenen Seren kultiviert.

In Franchimont et al. "Effects of hormones and drugs on cartilage repair" aus dem Journal of Rheumatology, 1989, Vol. 16, Seiten 5-9, ist die Suspensionskultivierung von Chondrozyten beschrieben.

Fritsch et al. "Advances in autologous chondrocyte transplantation (ACT)" Techvest's first annual conference on tissue repair, replacement and regeneration, Oktober 27-28, 1999,

New York beschreibt die Bildung von Knorpel-Cluster nach 4 Tagen Kultur und dass sich Chondrozyten innerhalb der ersten 15 Tage in Kultur vermehren.

Löhnert et al. "Autologe Chondozytentransplantation (ACT) im Kniegelenk; erste klinische Ergebnisse" aus Arthroskopie, Vol. 12, 1999, Seiten 34-42 beschreibt die Applikation von in vitro hergestellten Chondrozyten mittels einer dünnen Kanüle in den Defektbereich.

Gruber et al. "Untersuchung zur In-vitro Kultivierung von Humanchondrozyten bei Einsatz von autologem Humanserum als Mediumzusatz: Minimierung der möglichen Risikos einer Infektion mit Erregern von Prionen-Erkrankungen"; Laryngo-Rhino-Otol. 75 (1996) 105-108, beobachtet eine verbesserte Proliferationsverhalten bei mit Humanserum gezüchteten Humanchondrozyten unter Minimierung des Risikos einer Infektion durch Prionen.

Es wurde überraschenderweise gefunden, dass diese Aufgabe mit dem in Anspruch 1 angegebenen, einfachen Verfahren gelöst werden kann.

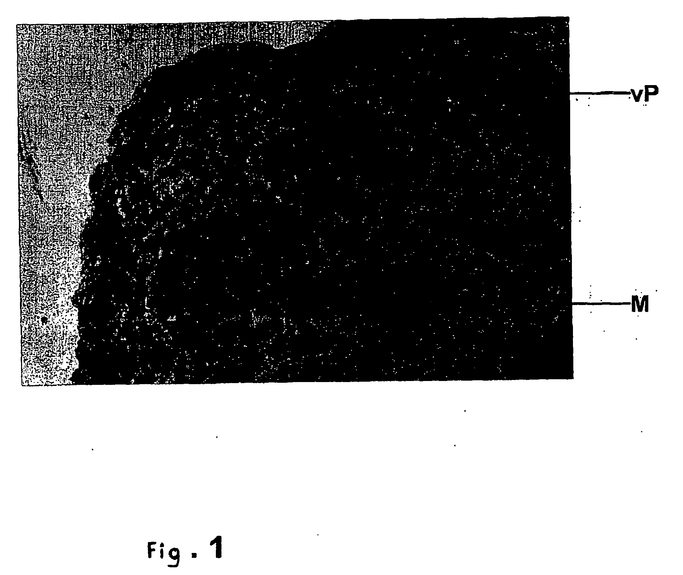

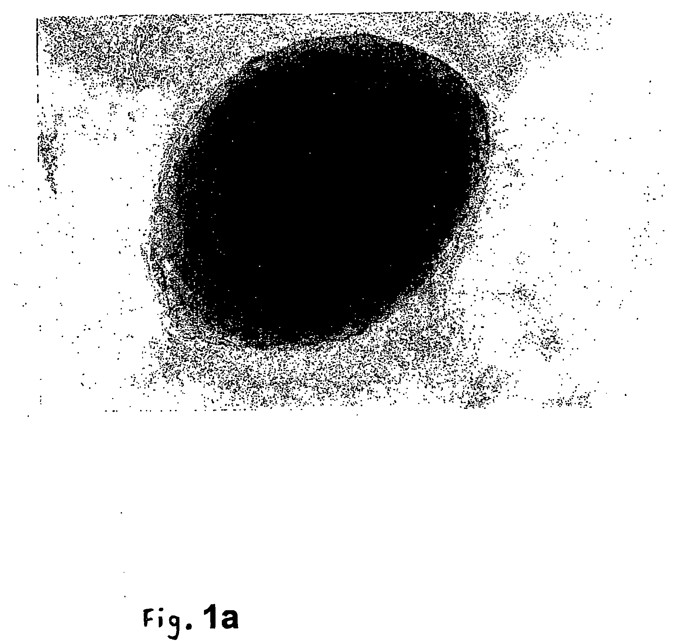

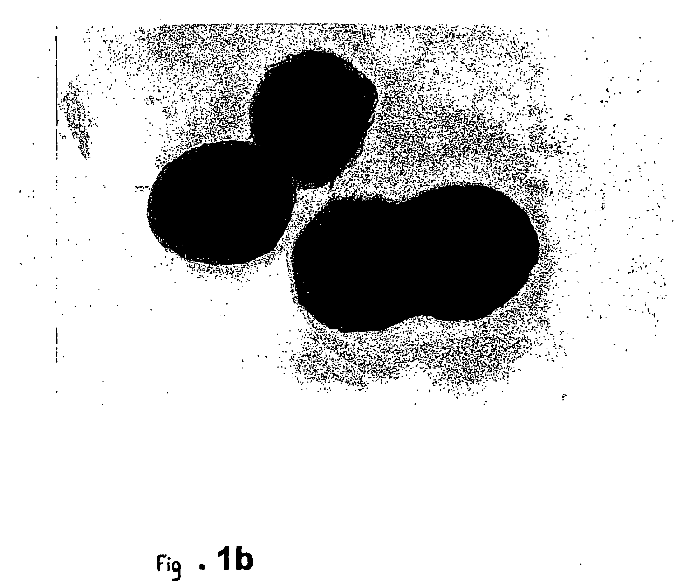

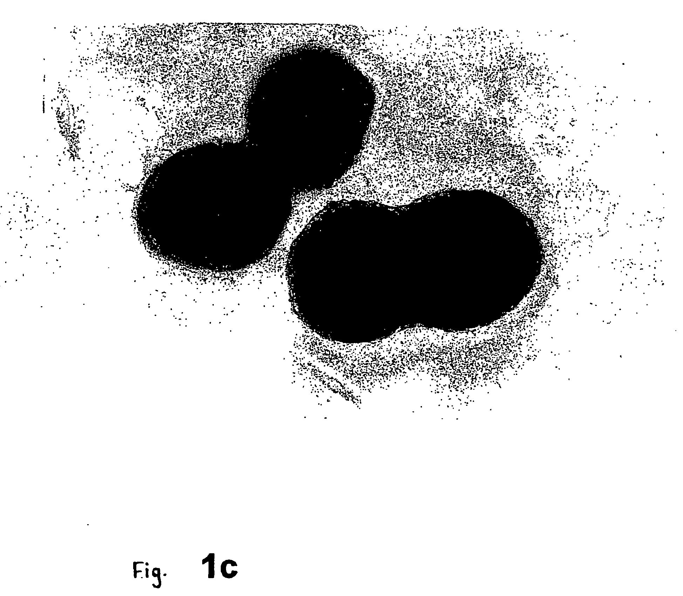

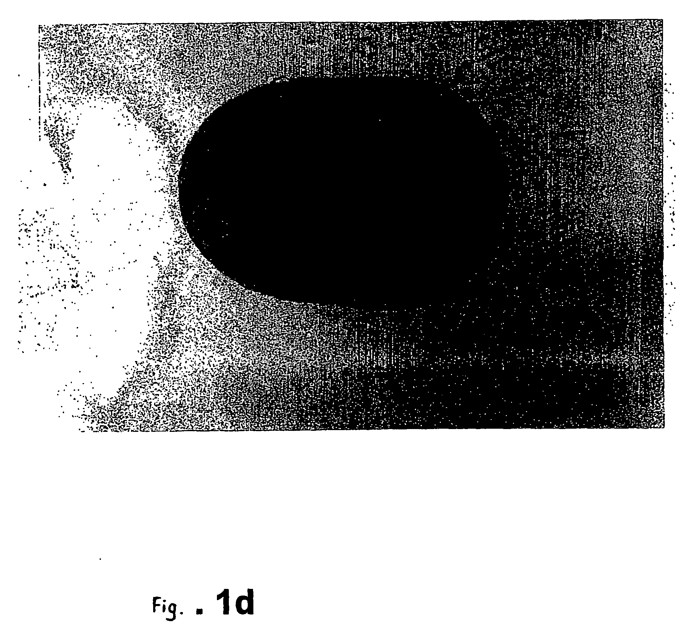

Erfindungsgemäß werden als Ausgangsmaterial patienteneigene Gewebebiopsien oder -proben oder mesenchymale Stammzellen, z.B. aus dem peripheren Blut oder Knochenmark verwendet. Aus den Biopsien werden die gewebeaufbauenden Zellen mittels enzymatischem Verdau des Gewebes, durch Auswandern oder durch Reagenzien, die Zielzellen erkennen, mit üblichen Methoden isoliert. Diese Zellen werden dann erfindungsgemäß in einfacher Weise mit üblichem Kulturmedium in Zellkulturgefäßen mit hydrophober Oberfläche und sich verjüngendem Boden in Suspension so lange stationär kultiviert, bis ein dreidimensionales Zellaggregat entsteht, das zu mindestens 40 Volumen%, vorzugsweise mindestens 60 Volumen% bis maximal 95 Volumen%, extrazelluläre Matrix (ECM) beinhaltet, in welche differenzierte Zellen eingebettet sind. Das entstandene Zellaggregat weist einen äußeren Bereich auf, in welchem proliferations- und migrationsfähige Zellen vorhanden sind. Diesen Aufbau der erfindungsgemäß erhaltenen Zellaggregate verdeutlichen die mikroskopischen Aufnahmen in Abb. 1 und la, wobei Abb. 1 die Ausschnittsvergrößerung des Querschnittes eines erfindungsgemäßen Zellaggregates mit vP als Zone des Auftretens der ersten gewebespezifischen Matrixproteine und M als Zone der Bildung von gewebespezifischen Matrixproteinen darstellt, und Abb. 1a das gesamte Zellaggregat mit der äußeren Zone proliferationsfähiger und migrationsfähiger Zellen P (Zone der Expression des Proteins S 100) zeigt.

Es ist erstaunlich, dass alle Zellen, die in den nach dieser Erfindung hergestellten Sphäroiden integriert sind, überleben und auch nach fortschreitender Kultivierungsdauer die Zellen im Inneren nicht absterben. Mit fortschreitender Kultivierungsdauer differenzieren die Zellen im Inneren der Aggregate aus und es bilden sich Sphäroide, die aus ECM, differenzierten Zellen und einer Proliferationszone am Rand bestehen. Der Prozess der Bildung dieser gewebespezifischen Matrix mit eingebetteten Zellen ist dem Prozess der Gewebsentstehung bzw. -neubildung und -umbildung im Körper sehr ähnlich. Während der Differenzierung in Zellkultur wird der Abstand der aggregierten Zellen durch Bildung der gewebespezifischen Matrix immer größer. Es entsteht im Inneren der Sphäroide eine Gewebehistologie, die dem natürlichen Gewebe sehr ähnlich ist. Die Versorgung der Zellen im Inneren der Sphäroide erfolgt, wie im natürlichen Knorpel auch, allein durch die Diffusion der Nährstoffe. Während der weiteren Herstellung der Sphäroide bildet sich die Zone proliferationsfähiger und migrationsfähiger Zellen am Rand der Sphäroide. Diese Zone hat den unschätzbaren Vorteil, dass nach Einbringen der Sphäroide in Defekte, die in dieser Randzone befindlichen Zellen in der Lage sind, auszuwandern und aktiv den Kontakt zum umliegenden Gewebe herzustellen bzw. eine Integration des in vitro gebildeten Gewebes in seine Umgebung ermöglichen. Damit sind die hergestellten gewebespezifischen Zellaggregate hervorragend zur Behandlung von Gewebsdefekten und zum Neuaufbau von Gewebe in vitro und *in vivo* geeignet.

In Abhängigkeit von der Größe des zu behandelnden Gewebedefektes kann es von Vorteil sein, bereits größere Gewebestücke zu transplantieren, um ein schnelleres Auffüllen des Defektes zu erreichen. Für diesen Fall werden mindestens zwei, besser aber mehr der erhaltenen Zellaggregate fusioniert, indem sie gemeinsam unter den gleichen Bedingungen und in den gleichen Kulturgefäßen wie oben beschrieben bis zur gewünschten Größe weiterkultiviert werden.

Abb. 1b zeigt zwei zu fusionierende Sphäroide nach einem Tag.

Abb. 1c zeigt, dass bereits einige Stunden später die Grenze zwischen den beiden Sphäroiden nicht mehr zu erkennen ist. Nach einer weiteren Woche sind die Sphäroide vollständig fusioniert und es ist ein größeres in vitro Gewebestück entstanden (Abb. 1d). Der Aufbau der so erhaltenen größeren Zellaggregate ist mit dem der zunächst erhaltenen Sphäroide identisch. Sie können bis zu maximal 95% ECM beinhalten und alle im erhaltenen Gewebestück enthaltenen Zellen sind vital.

Das erhaltene Knorpel- oder Knochengewebe ist außerordentlich stabil. Die Zellaggregate können auf ³/₄ ihres Durchmessers komprimiert werden, ohne dass sie zerbrechen oder beispielsweise beim Injizieren in den Körper mittels einer Kanüle auseinanderfallen. Es ist möglich, diese Gewebestückchen mit einer Pinzette oder einer Pipette aus dem Zellkulturgefäß zu entnehmen.

Um ausreichend Knorpel- oder Knochenzellen für die erfindungsgemäße Suspensionskultivierung zur Verfügung zu haben, werden die vom Patienten gewonnenen Zellen in einer vorteilhaften Ausführungsform der Erfindung zunächst in an sich bekannter Art und Weise in Monolayerkultur vermehrt. Die Passage der Zellen in Monolayerkultur wird so gering wie möglich gehalten. Nach Erreichen des konfluenten Stadiums werden die in Monolayer gewachsenen Zellen geerntet und gemäß des erfindungsgemäßen Verfahrens in Suspension, wie oben beschrieben, kultiviert.

Als Zellkulturmedium kann sowohl für die Suspensions- als auch für die Monolayerkultur übliches Medium, z.B. *Dulbecco's MEM,* unter Zusatz von Serum, verwendet werden. Vorzugsweise wird DMEM und Hams im Verhältnis 1:1 eingesetzt. Um jedoch immunologische Reaktionen des Patienten auf das in vitro hergestellte Gewebe zu vermeiden, wird als Serum vorzugsweise autogenes Serum des Patienten eingesetzt. Es ist auch möglich xenogenes oder allogenes Serum zu verwenden.

Dem Kulturmedium werden erfindungsgemäß keine Antibiotika, Fungistatika oder andere Hilfsstoffe zugesetzt. Es hat sich gezeigt, dass nur die autogene, xenogene oder allogene Kultivierung der Zellen und Zellaggregate sowie die Kultivierung ohne Antibiotika und Fungistatika eine unbeeinflußte Morphologie sowie Differenzierung der Zellen in der Monolayerkultur und eine ungestörte Bildung der spezifischen Matrix in den Zellaggregaten ermöglicht. Weiterhin sind durch den Verzicht sämtlicher Zusatzstoffe während der Herstellung nach Einbringen des in vitro hergestellten Gewebes in den menschlichen und auch tierischen Organismus sämtliche immunologische Reaktionen ausgeschlossen.

Es ist allerdings überraschend, dass weder bei der Suspensionskultivierung, noch bei der Monolayerkultivierung Wachstumsfaktoren oder andere wachstumsfördemde Zusätze notwendig sind. Trotz des Fehlens dieser Zusätze werden bereits nach zweitägiger erfindungsgemäßer Suspensionskultivierung dreidimensionale Zellaggregate mit gewebespezifischen Eigenschaften erhalten. Die Größe hängt natürlich von der eingebrachten Zellzahl pro Volumen Kulturmedium ab. Werden beispielsweise 1 x 10⁷ Zellen in 300 µl Kulturmedium eingebracht, so entstehen innerhalb von 1 Woche dreidimensionale Sphäroide von ca. 500-700 µm Durchmesser. Für einen 1 cm²-Gewebedefekt müssten ca. 100 solcher Sphäroide transplantiert, z.B. injiziert, werden. Die andere Möglichkeit ist die in vitro-Fusion der kleinen Zellaggregate zu größeren - wie oben beschrieben - und das Einbringen dieser in den Defekt. Vorzugsweise werden erfindungsgemäß zwischen 1 x 10⁴ und 1 x 10⁷ Zellen in 300 µl Kulturmedium zur Herstellung der kleinen Zellaggregate verwendet, besonders bevorzugt 1 x 10⁵ Zellen. Die nach einigen Tagen gebildeten Sphäroide werden dann für mindestens 2-4 Wochen in Abhängigkeit von der Zellart und den patientenspezifischen Charakteristika in dem geeigneten Kulturmedium kultiviert, um die Ausbildung der gewebespezifischen Matrix zu induzieren. Im besonderen Falle können dann einzelne Sphäroide ab ca. einer Woche Kultivierung fusioniert werden, um die Größe des Gewebestückes zu erhöhen.

Als Zellkulturgefäße müssen für die erfindungsgemäße Kultivierung in Suspension solche mit hydrophober, also adhäsionsverhindernder Oberfläche, wie z.B. Polystyrol oder Teflon, eingesetzt werden. Zellkulturgefäße mit nichthydrophober Oberfläche können durch Beschichten mit Agar oder Agarose hydrophobiert werden. Weitere Zusätze sind nicht erforderlich. Vorzugsweise dienen als Zellkulturgefäße Napfplatten. Dabei können für die Herstellung der kleinen Zellaggregate beispielsweise 96-Napfplatten und für die Herstellung der fusionierten Aggregate 24-Napfplatten Verwendung finden.

Erfindungsgemäß müssen die Zellkulturgefäße einen sich verjüngenden, vorzugsweise gewölbten Boden aufweisen. Es hat sich gezeigt, dass sich das erfindungs gemäße Gewebe in Gefäßen mit flachem Boden nicht bildet. Offensichtlich dient die Vertiefung zum Finden der Zellen.

Die vorliegende Erfindung offenbart auch das nach dem oben beschriebenen Verfahren hergestellte Knorpel- oder Knochengewebe, das als autogenes, xenogenes oder allogenes Transplantationsmaterial in der Behandlung von Knorpel- oder Knochendefekten und von degenerativen Erkrankungen wie z.B. Arthrose oder Rheuma Verwendung finden kann. Dazu werden die erfindungsgemäßen in vitro hergestellten Zellaggregate mittels Injektion in das erkrankte oder abgebaute Gewebe eingespritzt. Dazu muss die Injektionsnadel oder ein anderes geeignetes Applikationssystem mindestens den Durchmesser der Sphäroide haben. Die Zellen in der Zone proliferationsfähiger und migrationsfähiger Zellen am Rande der Sphäroide wachsen schnell in das umliegende Gewebe ein und ermöglichen eine schnelle Einbindung des in vitro hergestellten Gewebes und stellen ein Potential zur Neubildung von Gewebe in der Umgebung der Sphäroide dar, da diese Zellen noch proliferieren und Matrix bilden. Diese Behandlung kann im Fortschritt zu bisherigen Verfahren im Tissue Engineering arthroskopisch erfolgen.

Die vorliegende Erfindung offenbart auch therapeutische Zubereitungen, die das erfindungsgemäße Knorpel- oder Knochengewebe umfassen, z. B. Injektionslösungen.

Die vorliegende Erfindung offenbart auch die Verwendung des erfindungsgemäßen Knorpel- oder Knochengewebes zur Testung verschiedener Faktoren, wie z.B. Wirkstoffen und physikalischen Faktoren, die z.B. die Bildung und Differenzierung von Matrix und Zellen beeinflussen, wobei die physikalischen Faktoren z.B. Druck oder elektrische Felder sein können. Dazu werden die Zellsphäroide erfindungsgemäß hergestellt und in unterschiedlichen Reifestadien werden die zu testenden Medikamente hinzugegeben und unterschiedlichste Parameter der Sphäroidentstehung und -reifung charakterisiert. Diese Tests sind im Vergleich zu den herkömmlichen Medikamententests an Tieren oder Tumorsystemen durch die Verwendung von nur autologem Material sehr patientenspezifisch und ermöglichen eine individuelle Diagnose.

Nachfolgend soll die Erfindung an Ausführungsbeispielen näher erklärt werden, ohne sie darauf einzuschränken.

### Ausführungsbeispiele

### Beispiel 1: in vitro-Herstellung von Knorpelgewebe

Vom Patienten wird aus einem Bereich hyalinen, gesunden Knorpels eine Biopsie entnommen. Aus dieser Biopsie werden mittels enzymatischen Verdaus durch Inkubation mit Kollagenaselösung die Chondrozyten isoliert. Nach Trennung der isolierten Zellen vom unverdauten Knorpelgewebe, werden diese in Zellkulturflaschen überführt und unter Zugabe von DMEM / Hams F12 Kulturmedium (1/1) und 10% autologem Serum des Patienten bei 37°C und 5% CO₂ inkubiert. Zweimal wöchentlich wird ein Mediumwechsel durchgeführt. Nach Erreichen des konfluenten Stadiums wird die Zellayer mit physiologischer Kochsalzlösung gewaschen und mittels Trypsin von der Zellkulturoberfläche geerntet. Nach einer weiteren Waschung werden 1 x 10⁵ Zellen in je ein Zellkulturgefäß überführt, das mit Agarose beschichtet ist. Nach einem Tag haben sich die ersten Zellen in Aggregaten angeordnet. Diese Aggregate werden alle 2 Tage mit frischem Medium versorgt und für mindestens 2 Wochen kultiviert. Bereits nach einer Woche wurden Kollagen Typ II und Proteoglycane in den Aggregaten nachgewiesen. Dazu wurde ein spezifischer Antikörper gegen Kollagen Typ II verwendet. Der an Kollagen Typ II gebundene Primärantikörper wurde mit Hilfe eines Zweitantikörpers und daran gekoppeltem ABC-System nachgewiesen. Das heißt, an dem 2. Antikörper ist über Avidin-Biotin das Enzym Alkalische Phosphatase gekoppelt, welches das Substrat Fuchsin umsetzt, wobei ein roter Farbstoff entsteht.

Die Proteoglycane wurden mittels Goldnerfärbung nachgewiesen. Kollagen Typ II und Proteoglykane sind Bestandteile der Knorpelmatrix *in vivo* und stellen die wichtigsten Strukturproteine dar, die für die Funktion des Knorpels von entscheidender Bedeutung sind.

In der äußeren Schicht der Aggregate wurde zum gleichen Zeitpunkt das für Knorpelzellen spezifische Protein S 100 nachgewiesen. S 100 wird nicht in Knochengewebe und Bindegewebe exprimiert. Nur diese Gewebe könnten hierbei auch entstehen. Somit wurde eindeutig nachgewiesen, dass das entwickelte Gewebe Knorpelgewebe ist.

Nach 1-2 Wochen Kultivierung liegen die Zellen noch dicht beieinander. Mit steigender Kultivierungsdauer nimmt der Anteil an extrazellulärer Matrix zu und der Anteil an Zellen ab. Nach einer Woche ist mindestens 40% ECM nachweisbar und nach 3 Wochen wurde bereits ca. 60% ECM entwickelt. Nach 3 Monaten Kultivierung der Knorpelgewebe ist der Anteil an ECM auf 80-90% angestiegen. Das heißt, im Inneren der hergestellten Aggregate wurde knorpelartiges Gewebe aufgebaut, welches im Aufbau dem *in vivo* Knorpel entspricht und auch die Funktion von Knorpelgewebe übernehmen kann.

### Beispiel 2: Transplantation von Knorpelgewebe

Das in Beispiel 1 hergestellte Gewebe (ca. 200 Sphäroide aus je 1*10⁵ Zellen) wurde in physiologischer Kochsalzlösung aufgenommen und in einen ca. 1 cm² großen, mit Periost abgedeckten Knorpeldefekt des Probanden eingespritzt. Es wurde festgestellt, dass der Knorpeldefekt bereits innerhalb von 1 bis 2 Monaten mit Knorpelgewebe aufgefüllt ist, während durch die bisherige Transplantation von jungen, lediglich in vitro vermehrten Knorpelzellen die Auffüllung eines Defektes einer solchen Größe erst nach 6-12 Monaten zu verzeichnen ist. Das erfindungsgemäße in vitro hergestellte Knorpelgewebe gewährleistet also neben der Erfüllung der mechanischen Funktion des hergestellten Gewebes die rasche Integration der hergestellten Gewebestücke durch die proliferationsfähigen und migrationsfähigen Zellen in der äußeren Schicht der Aggregate. Somit erlaubt die Struktur und Funktion der Gewebestücke auch die schnelle Reparatur von Defekten und degeneriertem Knorpelgewebe.

### Beispiel 3: in vitro-Herstellung von Knochengewebe

Vom Patienten wird eine Knochenbiopsie aus dem Bereich des Spongiosaknochens entnommen. Aus dieser Biopsie werden mittels enzymatischen Verdaus durch Inkubation mit Kollagenaselösung die Osteoblasten isoliert. Nach Trennung der isolierten Zellen vom unverdauten Knochengewebe, werden diese in Zellkulturflaschen überführt und unter Zugabe von DMEM / Hams F12 Kulturmedium (1/1) und 10% autologem Serum des Patienten bei 37°C und 5% CO₂ inkubiert. Zweimal wöchentlich wird ein Mediumwechsel vorgenommen. Nach Erreichen des konfluenten Stadiums wird die Zellayer mit physiologischer Kochsalzlösung gewaschen und mittels Trypsin von der Zellkulturoberfläche geerntet. Nach einer weiteren Waschung werden 1 x 10⁵ Zellen in je ein Zellkulturgefäß überführt, das mit Agarose beschichtet ist. Nach einem Tag haben sich die ersten Zellen in Aggregaten angeordnet. Diese Aggregate werden alle 2 Tage mit frischem Medium versorgt und für mindestens 2 Wochen kultiviert.

Bereits nach einer Woche wurden Kollagen Typ I und Proteoglycane in den Aggregaten nachgewiesen. Dazu wurde ein spezifischer Antikörper gegen Kollagen Typ I verwendet. Durch den Nachweis von Kollagen I wurde zweifelsfrei nachgewiesen, dass es sich nicht um Knorpelgewebe handelt. Der an Kollagen Typ I gebundene Primärantikörper wurde mit Hilfe eines Zweitantikörpers und daran gekoppeltem ABC-System nachgewiesen. Das heißt, an dem 2. Antikörper ist über Avidin-Biotin das Enzym Alkalische Phosphatase gekoppelt, welches das Substrat Fuchsin umsetzt, wobei ein roter Farbstoff entsteht.

Die Proteoglycane wurden wie in Beispiel 1 mittels Goldnerfärbung nachgewiesen. Kollagen Typ I und Proteoglykane sind Bestandteile der Knochenmatrix *in vivo* und stellen die wichtigsten Strukturproteine dar, die für die Funktion des Knochens von entscheidender Bedeutung sind.

In der äußeren Schicht der Aggregate wurden zum gleichen Zeitpunkt proliferationsfähige Knochenzellen nachgewiesen.

Nach 2 Wochen Kultivierung liegen die Zellen noch dicht beieinander. Mit steigender Kultivierungsdauer nimmt der Anteil an extrazellulärer Matrix zu und der Anteil an Zellen ab. Nach einer Woche ist mindestens 40% ECM nachweisbar und nach 3 Wochen wurde bereits ca. 60% ECM entwickelt. Das heißt, im Inneren der hergestellten Aggregate wurde knochenartiges Gewebe aufgebaut, welches in der Zusammensetzung dem *in vivo* Knochen entspricht und auch die Funktion von Knochengewebe übernehmen kann.

### Beispiel 4: Transplantation von Knochengewebe

Die in Beispiel 3 hergestellten Gewebe (ca. 50 Stück) werden in physiologischer Kochsalzlösung aufgenommen und in den Bereich einer schwer heilenden Knochenfraktur gespritzt. Es wurde festgestellt, dass der Prozess der Knochenheilung induziert werden konnte und bereits nach 3 Wochen neues Knochengewebe gebildet wurde, während bei ausbleibender Behandlung des Defektes eine Bruchheilung erst nach Monaten erfolgt wäre. Das in vitro gezüchtete Knochengewebe gewährleistet also die schnelle Integration des Gewebes in das umliegende Gewebe und die Auffüllung von Knochendefekten. Somit erlaubt die Struktur und Funktion der Gewebestücke die Heilung von Knochendefekten, die Induktion der Osteosynthese und die Behandlung von degenerativen Knochenerkrankungen.

## Patentansprüche

1. Verfahren zur in vitro-Herstellung von einem Zellaggregat aus dreidimensionalem, vitalem Knorpel- oder Knochengewebe, welches ein Sphäroid ist, **dadurch gekennzeichnet, dass** aus einem menschlichen oder tierischen Organismus gewonnene Knorpelzellen, Knochenzellen, oder mesenchymale Stammzellen in Zellkulturgefäßen mit hydrophober Oberfläche und sich verjüngendem Boden als Suspensionskultur unter Zusatz von autogenem Serum oder autologem Serum und ohne Wachstumsfaktoren und ohne Antibiotika und ohne Fungistatika stationär so lange kultiviert werden bis ein Zellaggregat entsteht, welches ein Sphäroid ist und das zu mindestens 40 Vol. % extrazelluläre Matrix (ECM) beinhaltet, in welche differenzierte Zellen eingebettet sind, und das einen äußeren Bereich aufweist, in welchem proliferations- und migrationsfähige Zellen vorhanden sind.

2. Verfahren nach Anspruch 1, **dadurch gekennzeichnet, dass** die aus dem Explantat gewonnenen Knorpel- oder Knochenzellen zunächst in Monolayerkultur vennehrt werden und anschließend die Kultivierung in Suspension gemäß Anspruch 1 vorgenommen wird.

3. Verfahren nach einem der Ansprüche 1 bis 2, **dadurch gekennzeichnet, dass** je nach gewünschter Gewebegröße mindestens zwei der entstandenen Zellaggregate, welche Sphäroide sind, fusioniert werden, indem sie gemeinsam in Zellkulturgefäßen mit hydrophober Oberfläche und sich verjüngendem Boden in Suspension weiterkultiviert werden.

4. Verfahren nach einem der Ansprüche 1 bis 3, **dadurch gekennzeichnet, daß** als Zellkulturgefäße Napfplatten eingesetzt werden.

5. Verfahren nach einem der Ansprüche 1 bis 4, **dadurch gekennzeichnet, daß** Zellkulturgefäße mit nichthydrophober Oberfläche durch Beschichten mit Agar oder Agarose hydrophobiert werden.

6. Verfahren nach einem der Ansprüche 1 bis 5, **dadurch gekennzeichnet, daß** die Kultivierung in Suspensionskultur so lange durchgeführt wird, bis das erhaltene Zellaggregat mindestens 60% ECM aufweist.

## Claims

1. A method for in vitro production of a cell aggregate of three-dimensional vital cartilage or bone tissue in a spheroid shape, **characterized in that** cartilage cells, bone cells or mesenchymal stem cells derived from human or animal organisms are cultivated in a stationary manner as a suspension culture in cell culture vessels with hydrophobic surfaces and tapered base, with the addition of autogenous serum or autologous serum and without growth factors and without antibiotics and no fungistatics, until a cell aggregate results that is spheroid in shape and which contains at least 40 % by volume of extracellular matrix (ECM) in which differentiated cells are imbedded and having an outer area in which proliferation- and migration-capable cells are present.

2. The method according to Claim 1, **characterized in that** the cartilage or bone cells derived from the explants are initially multiplied in monolayer culture before being cultivated in suspension according to Claim 1.

3. The method according to either of Claims 1 or 2, **characterized in that**, depending on the desired tissue size, at least two of the resulting spheroid cell aggregates are fused by being further cultivated together in suspension in cell culture vessels with hydrophobic surfaces and tapered base.

4. The method according to any of Claims 1 to 3, **characterized in that** well plates are used as cell culture vessels.

5. The method according to any of Claims 1 to 4, **characterized in that** cell culture vessels with non-hydrophobic surface are made hydrophobic by coating with agar or agarose.

6. The method according to any of Claims 1 to 5, **characterized in that** cultivation in suspension culture is performed until the resulting cell aggregate contains at least 60 % ECM.

## Revendications

1. Procédé de production in vitro d'un agrégat cellulaire de tissu vital tridimensionnel cartilagineux ou osseux, qui est sphéroïde, **caractérisé en ce que** lesdites cellules cartilagineuses, osseuses ou cellules souches mésenchymateuses provenant d'organismes humains ou animaux sont cultivées de façon stationnaire en suspension dans des récipients de culture cellulaire ayant une surface hydrophobe et une base effilée, avec addition de sérum autogène ou de sérum autologue, sans facteurs de croissance, antibiotiques ou fongistatiques, jusqu'à obtention d'un agrégat cellulaire sphéroïdal contenant au moins 40 % du volume de la matrice extracellulaire (MEC) dans laquelle les cellules différenciées sont intégrées, et présentant une zone externe sur laquelle se trouvent des cellules capables de prolifération et de migration.

2. Procédé selon la revendication 1, **caractérisé en ce que** les cellules cartilagineuses ou osseuses provenant des explants sont d'abord multipliées en culture monocouche avant d'être cultivées en suspension selon la revendication 1.

3. Procédé selon la revendication 1 ou 2, **caractérisé en ce qu'**en fonction de la taille de tissu désirée, deux ou plusieurs des agrégats cellulaires sphéroïdaux obtenus sont fusionnés par culture associée supplémentaire en suspension dans des récipients de culture cellulaire ayant une surface hydrophobe et une base effilée.

4. Procédé selon une quelconque des revendications 1 à 3, **caractérisé en ce que** des plaques à puits servent de récipients de culture cellulaire.

5. Procédé selon une quelconque des revendications 1 à 4, **caractérisé en ce que** les récipients de culture cellulaire ayant une surface non hydrophobe sont rendus hydrophobes par revêtement avec l'agar-agar ou l'agarose.

6. Procédé selon une quelconque des revendications 1 à 5, **caractérisé en ce que** la culture en suspension est effectuée jusqu'à obtention d'un agrégat cellulaire contenant au moins 60 % de MEC.
